(19) Europäisches Patentamt / European Patent Office / Office européen des brevets

(11) **EP 4 463 056 B1**

(12) **EUROPEAN PATENT SPECIFICATION**

(45) Date of publication and mention
of the grant of the patent:
**10.12.2025 Bulletin 2025/50**

(21) Application number: **22850607.7**

(22) Date of filing: **30.12.2022**

(51) International Patent Classification (IPC):
**A61B 3/16** *(2006.01)*

(52) Cooperative Patent Classification (CPC):
**A61B 3/16**

(86) International application number:
**PCT/IB2022/062926**

(87) International publication number:
**WO 2023/135484 (20.07.2023 Gazette 2023/29)**

(54) **A HEAD FOR THE GOLDMANN APPLANATION TONOMETER, A METHOD OF MEASURING AN INTRAOCULAR PRESSURE AND A METHOD OF IN VIVO MEASUREMENT OF A MODULUS OF ELASTICITY OF A CORNEA**

KOPF FÜR GOLDMANN-APPLANATIONSTONOMETER, VERFAHREN ZUR MESSUNG EINES AUGENINNENDRUCKS UND VERFAHREN ZUR IN-VIVO-MESSUNG EINES ELASTIZITÄTSMODULS EINER HORNHAUT

TÊTE POUR TONOMÈTRE À APPLANATION DE GOLDMANN, PROCÉDÉ DE MESURE DE PRESSION INTRAOCULAIRE ET PROCÉDÉ DE MESURE IN VIVO DE MODULE D'ÉLASTICITÉ D'UNE CORNÉE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **11.01.2022 IT 202200000278**

(43) Date of publication of application:
**20.11.2024 Bulletin 2024/47**

(73) Proprietor: **Iromed Group S.r.l.**
**00144 Roma (RM) (IT)**

(72) Inventors:
• **CARUSO, Ciro**
**80124 Napoli NA (IT)**
• **COSTAGLIOLA, Ciro**
**80122 Napoli NA (IT)**

(74) Representative: **Barbaro, Gaetano et al**
**Società Italiana Brevetti S.p.A.**
**Via G. Carducci, 8**
**20123 Milano (IT)**

(56) References cited:
**WO-A1-2021/205310     US-A- 5 305 747**

**Description**

TECHNICAL FIELD

This disclosure relates to tonometers for measuring intraocular pressure and more particularly to a head for the Goldmann applanation tonometer, to a method for measuring the intraocular pressure of a human or animal eye, and to a method for in vivo measurement of the modulus of elasticity of a cornea.

BACKGROUND

The measurement of intraocular pressure (IOP: IntraOcular Pressure) represents a fundamental moment of the routine ophthalmological examination, as the increase in its value represents the greatest risk factor for the onset of glaucoma and one of the fundamental parameters for the diagnosis of this disease, along with optic disc evaluation and visual field testing. Furthermore, the measurement of IOP provides useful indications for other ocular pathologies, such as uveitis, and in all patients undergoing eye surgery, and it is considered a routine examination to be performed in all ophthalmological visits.

The measurement of intraocular pressure can be performed with direct (manometry) or indirect (tonometry) methods. Ocular manometry is not used in clinical practice as it is more invasive, but is limited only to experimental use. Instead, ocular tonometry is the method most commonly used in clinical practice. It allows indirect measurement of intraocular pressure using instruments called tonometers, whose principle is based on the relationship between intraocular pressure and the force necessary to change the natural shape of the cornea.

In the following, reference will be made only to the "applanation" tonometers, which measure the force necessary to flatten a known surface area of the cornea or evaluate the size of the area flattened by a pre-established fixed force, as it is to them that the invention is applied.

The term "applanation tonometry", in the following description and in the claims, is intended to indicate any tonometer which exploits the principles of applanation tonometry to determine the intraocular pressure.

Applanation tonometry is based on the fact that, to flatten a surface of area A of the cornea, an average force F is required which acts on the surface of area A in order to balance the intraocular pressure (IOP):

$$IOP \text{ (Intra Ocular Pressure)} = F/A$$

It follows that the pressure inside an ideal sphere can be known by evaluating the width of the corneal area smoothed out by a constant force or by evaluating the force necessary to smooth out a known corneal area. Therefore, two different types of tonometers can be used for applanation tonometry: variable area tonometers and variable force tonometers.

The widest known and most used applanation tonometers in the world are those with variable force and undoubtedly the most famous of these is the Goldmann tonometer, now universally used, which represents the international standard for measuring IOP.

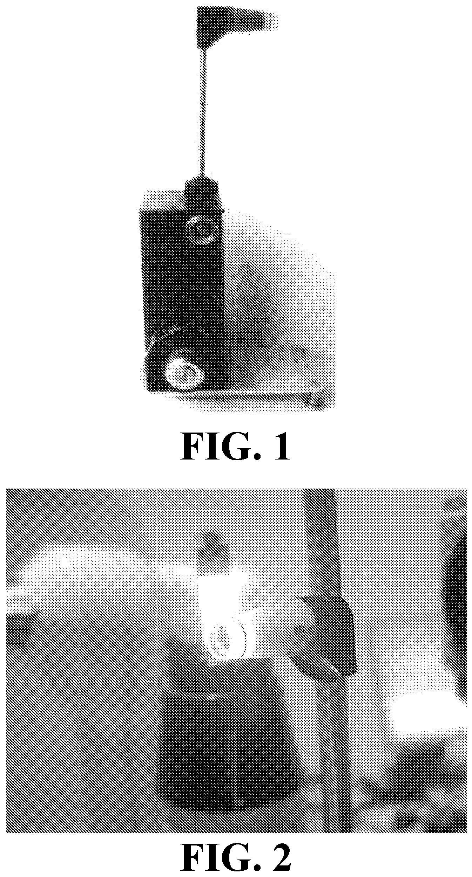

The Goldmann tonometer, represented in figure 1, comprises a smoothing element, consisting of a head typically made of transparent plastic or glass and containing a prism, joined by means of a sealing ring on a rod to a spiral spring. By means of a lateral graduated knob it is possible to vary the value of the force applied on the cornea, which is expressed in millimeters of mercury (mmHg) and is indicated by notches engraved on the knob itself.

The value of the flattened central cornea area is 3.06 mm$^2$ and this value, always constant, is set as the standard reference value.

The examination technique involves placing the instrument on a special base of a slit lamp, represented in figure 2. After corneal anesthesia, a coloring substance is applied to the patient's lower conjunctival arch on the anterior ocular surface, the coloring substance being a fluorescent substance that serves to make the edge of the flattened area more evident by observing with the cobalt blue light supplied with the slit lamp. As is known, identifying the edges of the flattened area with good precision is essential for a correct measurement of ocular pressure.

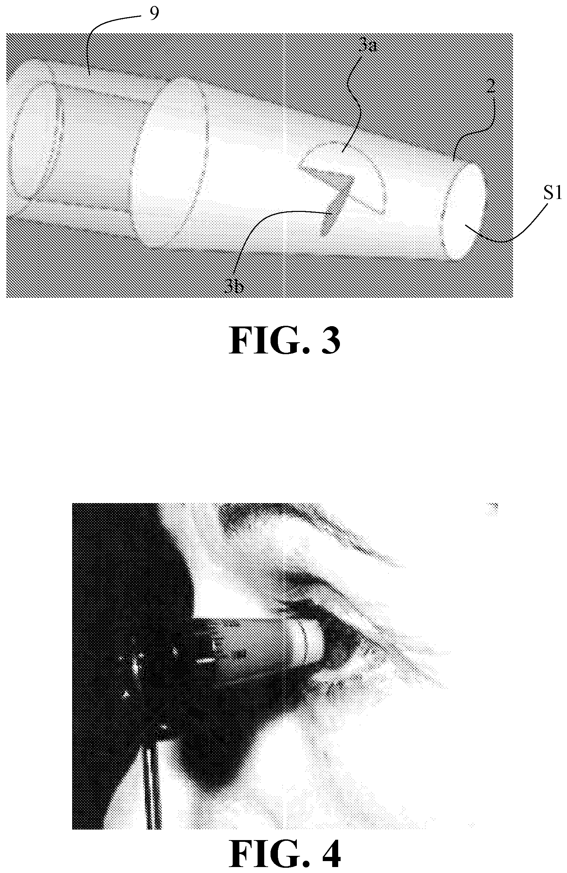

Figure 3 is a perspective view of a conventional applanation tonometer head. It appears externally as an object with substantially rotational symmetry along a longitudinal axial direction and has an integral body defining:

at a first end along the longitudinal axial direction, an end portion 2 defining a flat corneal applanation surface S1, having a respective applanation radius, arranged on a first base plane of the end portion 2 orthogonal to a longitudinal axis of the tonometer head;
two semi-cylindrical prisms 3a and 3b integral with the end portion 2, each having:

- a respective lower semicircular base arranged along a respective plane orthogonal to the longitudinal axial direction;
- a nominal height at a center of the respective lower semicircular base;
- a semi-elliptical upper base surface opposite the respective lower base and inclined with respect to it by a respective internal angle, typically between 8° and 60°.

The two semi-cylindrical prisms 3a and 3b are opposite each other in the height direction along a plane of axial separation so that the respective lower semi-circular bases lie in the same orthogonal plane together composing a full circle.

The known head of figure 3 typically has a tubular shank 7 which surrounds the upper base surfaces of the two prisms 3a and 3b, shown in semi-transparency, and is configured to be fitted into a sealing ring of an applanation tonometer while maintaining the cantilevered tonometer head outside the sealing ring, as shown in figure 2. The side surface of the current tonometer heads is opaque, so as to shield the prisms 3a and 3b from spurious light rays coming from the side and which could disturb the vision, and notches are made thereon to correctly align the separation plane of the prisms 3a and 3b to the patient.

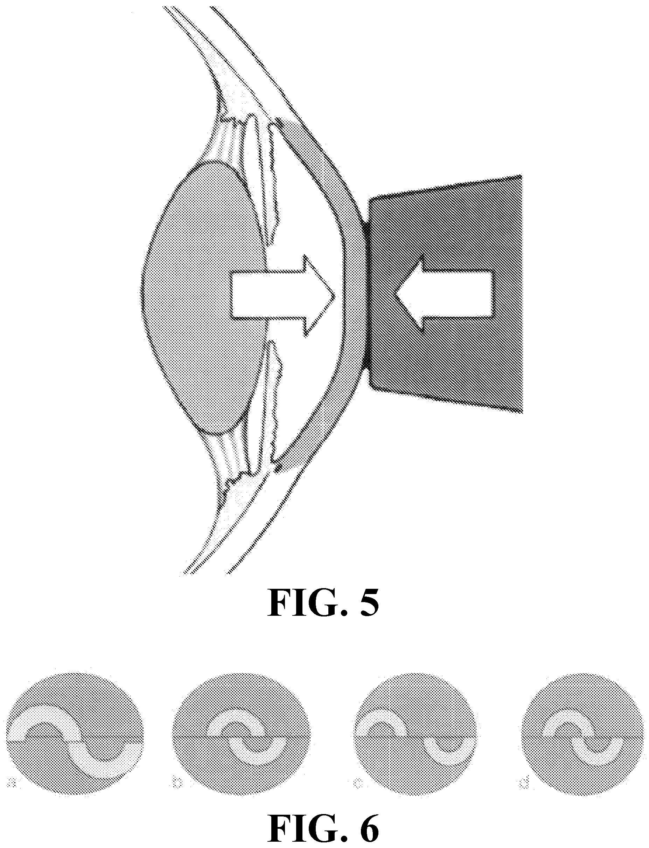

The patient is made to sit in front of the slit lamp and look at a point of reference, as illustrated in figure 4. The slit of the lamp is opened to the maximum and a cobalt blue filter is interposed to better visualize the ophthalmic dye, i.e. to better visualize the edges of the flattened area. A light beam is then directed so that it passes through the transparent prism and the knob is adjusted to the notch corresponding to 10 mmHg. The slit lamp is moved forward so that the prism gently contacts the central part of the cornea, as in figure 5.

Prisms 3a and 3b, contained in the transparent plastic head, split the circular image of the flattened corneal surface into two semicircles, one overlapped to the other. Then through the eyepiece, two green semicircles can be observed in a blue light field.

Figures 6a-6d show what is observed with the Goldmann tonometer in different cases. The lateral knob of the tonometer is rotated so as to bring the two semicircles into contact with their inner edge. Depending on the exerted pressure, different aspects of the semicircles can be observed: if these are far away, it is necessary to increase the applanation force; if they overlap, turn the knob in the opposite direction to decrease the exerted force. In Figure 6a, the hemicorons appear relatively thick due to excessive use of dye; in figure 6b the hemicrowns have an adequate thickness but are not correctly aligned because the applanation force is excessive; in figure 6c instead the applanation force is insufficient; in figure 6d the hemicrowns are correctly aligned. At this point, the amount of IOP indicated by the value on the knob is determined.

The measurement carried out with the tonometer is influenced by the physical characteristics of the cornea, such as its thickness, its modulus of elasticity, as well as by the attraction caused by the surface tension of the tear film, and in general it does not coincide with the intraocular pressure actually present in the eye, measurable with a manometric technique by inserting a probe through the cornea. According to Goldmann, inventor of the tonometer that bears his name, the effects of these physical characteristics compensate for the attraction due to surface tension when a corneal surface with a diameter of 3.06 mm is flattened. Under such conditions, the pressure measured by the Goldmann tonometer would correspond to the intraocular pressure IOP.

Several scholars have found that this is not strictly true and various formulas have been proposed in the literature to correct the readings provided by the Goldmann tonometer, in order to extrapolate the "true" IOP value. Some of these formulas do not take into account the modulus of elasticity of the cornea; other corrective formulas, very complex to use, are based on a hypothetical standard value of the modulus of elasticity. Unfortunately, the modulus of elasticity of an elderly patient's cornea can even be twice that of a healthy young patient, so corrective formulas may introduce further inaccuracies, rather than improve accuracy.

WO 2021/205310 discloses an applanation tonometer head with improved illumination comprising a transparent body having a longitudinal axis, an end portion defining a flat circular or elliptical corneal applanation surface, and two semi-cylindrical or semi-elliptical prisms each having lower semicircular or semi-elliptical bases and first semi-elliptical upper base surfaces inclined at angles between 10° and 60°. The prisms are positioned opposite along the longitudinal axis with respect to an axial separation plane. This document focuses on solving illumination problems in tonometer heads by providing better light transmission to the corneal applanation surface.

US 5,305,747 discloses optical elements for applanation tonometers comprising a tubular carrier and an optical element releasably held therein. The optical element includes a cylindrical body with a flat front face and a split field arrangement of prisms at the rear end. This document addresses hygiene concerns by providing replaceable/disposable optical elements but uses conventional prism arrangements without the specific geometric configurations of the present application.

SUMMARY

Studies performed by the Applicant have shown that the elastic reaction force exerted by a flattened cornea against the applanation surface of the head of a Goldmann tonometer can be estimated by knowing the modulus of elasticity of the cornea, the central corneal thickness, the radius of the flattened corneal surface and the mean central radius of curvature of the cornea, given by the arithmetic mean between the inner central radius of curvature and the outer central radius of curvature of the cornea.

By appropriately shaping the prisms of a Goldmann tonometer head, as indicated in claim 1, it becomes possible to carry out in an accurately repeatable manner two different pressure measurements with two different radii of the flattened corneal surface. From these two different measurements, with a method according to the present disclosure it is possible to obtain the value of the "true" intraocular pressure of the patient, and therefore to correct the classic pressure measurement taken with the Goldmann tonometer. Furthermore, by knowing the central corneal thickness with any pachymeter and an average radius of curvature at the corneal apex with any keratometer, with another method according to the present disclosure it is possible to calculate in vivo the modulus of elasticity of the cornea of a patient according to the two different pressure measurements.

The methods of measuring the intraocular pressure and the modulus of elasticity in vivo are defined in claims 7 and 8. Other embodiments are defined in the appended claims.

BRIEF DESCRIPTION OF THE DRAWINGS

Figure 1 shows a Goldmann tonometer.

Figure 2 shows a classic head mounted in the holding ring of an applanation tonometer, illuminated by a blue light beam from a slit lamp.

Figure 3 is a perspective view of a conventional applanation tonometer head.

Figure 4 shows the familiar Goldmann tonometer head applied to a patient's eye;

Figure 5 illustrates the principle of measuring intraocular pressure with the Goldmann tonometer.

Figure 6 shows semicircles or hemicorons visualized with the Goldmann tonometer, in the following cases: a) excessive use of dye; b) excessive applanation force; c) insufficient applanation force; d) correctly aligned hemicorons.

Figure 7 shows a tonometer head according to the present disclosure, with an open rear tang.

Figure 8 shows another tonometer head according to the present disclosure, with a rear tang closed by a transparent surface.

Figure 9 is a sectional view of the tonometer head of Figure 8 along a longitudinal plane of separation of the two prisms of the head.

Figure 10 is a perspective view of the tonometer heads of Figures 7 and 8 sectioned along the XY plane to highlight the two prisms, each having three upper base surfaces.

Figure 11 is a plan view of the tonometer heads of Figures 7 and 8 sectioned along the XY plane to highlight the two prisms, having straight edges in pairs parallel.

Figure 12A is a sectional view along the XZ plane of the tonometer head of Figure 7 with the directions of propagation through the two prisms of the light rays generated by an annulus of ophthalmic dye having a radius equal to $g_1$, according to a measurement method of intraocular pressure according to this disclosure.

Figure 12B is a sectional view along the XZ plane of the tonometer head of Figure 7 with the directions of propagation through the two prisms of the light rays generated by an annulus of ophthalmic dye having a radius equal to $g_0$, according to the classical method of Goldmann measuring intraocular pressure.

Fig. 13A shows the image that the prisms of a tonometer head of this disclosure form when flattening a circular corneal surface of radius $g_1$ according to a method of measuring intraocular pressure according to this disclosure.

Fig. 13B shows the image that the prisms of a tonometer head of this disclosure form when a circular corneal surface of radius $g_0$ is flattened according to Goldmann's classic method of measuring intraocular pressure.

DESCRIPTION OF EXEMPLARY EMBODIMENTS

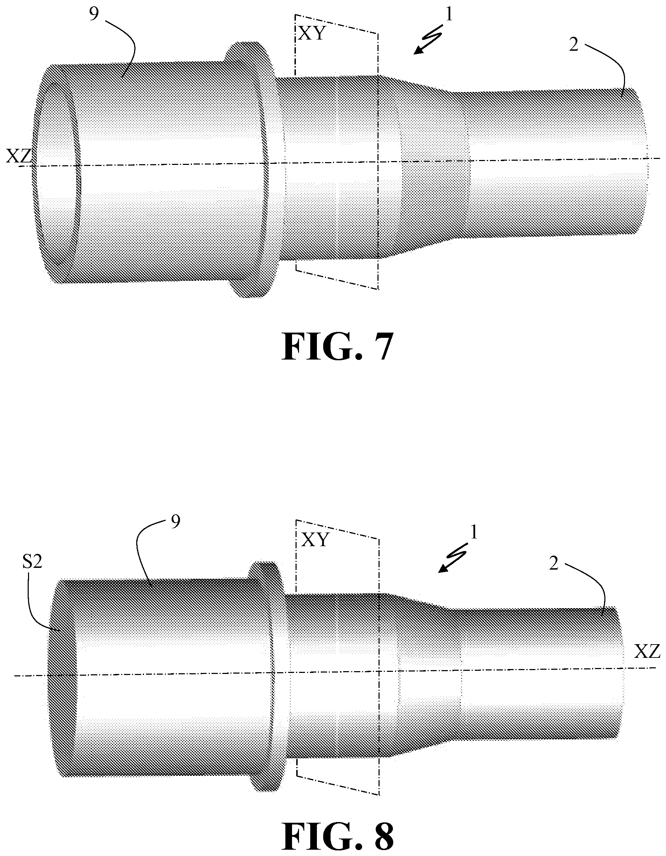

Some heads 1 for applanation tonometers according to the present disclosure are shown in figure 7, with rear tang 9 open, and in figure 8, with rear tang 9 closed by a transparent surface S2.

The embodiment of figure 7 can be more easily made by injection molding of transparent plastic material, such as for example PMMA or ABS, since it does not provide any closed cavity. Typically, disposable-type tonometer heads are made this way because they can be made in a single thermoplastic molding operation. Otherwise, the embodiment of figure 8 will be preferable for reusable tonometer heads, for example made of glass, which conveniently do not have open cavities, which are difficult to sterilize and dry, but have a transparent rear cover S2 which closes the internal cavity sealing it and preventing liquids or dust from entering it.

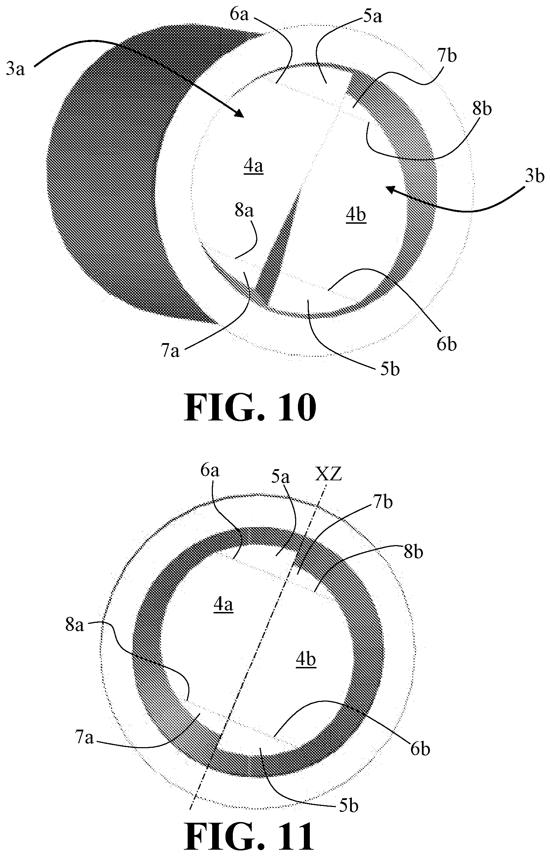

Like common applanation tonometer heads, a head 1 according to the present disclosure has a transparent body having a longitudinal axis Z, defining, at a first end along the longitudinal axis Z, an end portion 2 which has a surface circular or elliptical corneal applanation plane S1, having a respective radius or semi-axis of applanation, arranged on a first base plane of the end portion 2 orthogonal to the longitudinal axis Z of the tonometer head. In the central part of the head 1, two semi-cylindrical or semi-elliptical prisms 3a and 3b are defined, visible in figures 10 and 11 which represent, respectively in perspective and in plan view, a section of the head of figure 7 or of figure 8 along the XY plane. The two prisms 3a, 3b are integral with each other and with the end portion 2 and each of them has:

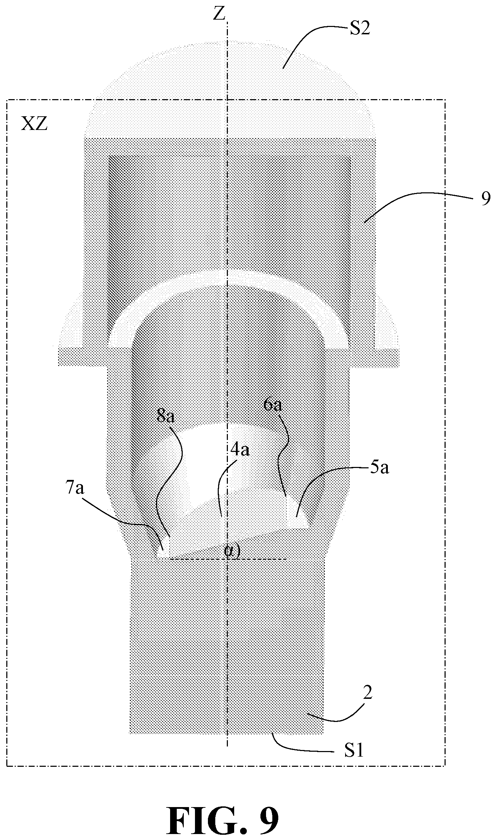

- a respective lower semicircular or semielliptical base integral with said first end and arranged along a second plane orthogonal to the longitudinal axis Z and parallel to the flat corneal applanation surface S1;
- a first semi-elliptical upper base surface 4a opposite the respective lower base and inclined with respect to the lower base by a respective internal angle $\alpha$ comprised between 9° and 60°, highlighted in the sectional view of figure 9 along the plane XZ.

As shown in figures 10 and 11, the two semi-cylindrical or semi-elliptical prisms 3a, 3b are opposite along the longitudinal axis with respect to the axial separation plane XZ so that the respective lower semi-circular or semi-elliptical bases are coplanar and lie in the second plane orthogonal by composing together a filled circle or a filled ellipse, respectively.

A particularity of the tonometer heads 1 of this disclosure consists in the fact that each of the two prisms is not defined only by an inclined upper surface 4a, as in the prisms of the classic head of figure 3, but has a profile in the shape of a broken line, clearly visible in figure 9. More specifically, each of the two prisms 3a, 3b defines at least a second upper base surface 5a, 5b parallel to the flat circular or elliptical corneal applanation surface S1 and bordering the first upper base surface 4a, 4b without gaps at a respective first straight edge 6a, 6b, which is at a first distance (measured in the axial separation plane XZ) from the longitudinal axis Z.

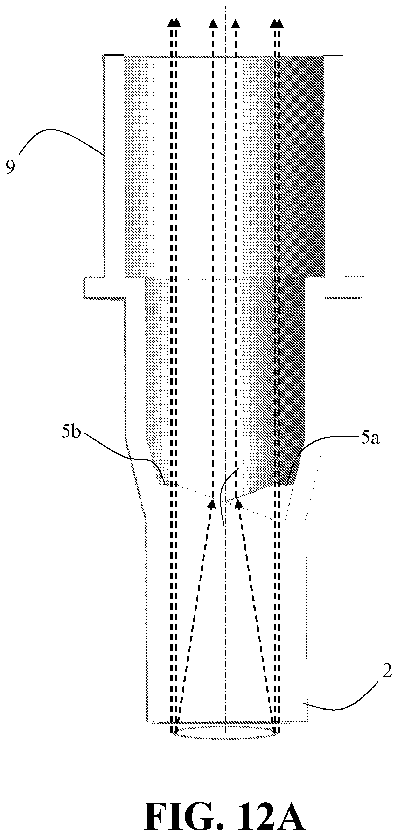

In practice, each of the two prisms 3a and 3b has at least a first inclined upper base surface 4a, 4b and a second upper base surface 5a, 5b parallel to the applanation surface S1. Thanks to this configuration of the second upper base surface 5a, 5b, a ray of light entering the end portion 2 from a point of the flat circular or elliptical corneal applanation surface S1 at a distance from the longitudinal axis Z equal to or greater than the first distance and which is directed along the axial separation plane XZ parallel to the longitudinal axis Z, crosses the end portion 2 and protrudes from the second upper base surface 5a always remaining directed parallel to the longitudinal axis, as schematically shown in figure 12A.

Each first straight edge 6a, 6b is at a first distance from the longitudinal axis Z and this first distance is between 1.6 mm and 3 mm. As will appear more clearly below, the presence of the first straight edge 6a, 6b and the value of this first distance are important to allow an accurate measurement of the intraocular pressure and to allow an in vivo estimate of the value of the modulus of elasticity E of the cornea of a patient.

Figures 9 to 14 refer to a preferred configuration in which each of the two prisms 3a, 3b, in addition to having a first inclined upper base surface 4a, 4b and a second upper base surface 5a, 5b parallel to the applanation surface S1, also has an optional third upper base surface 7a, 7b, parallel to the flat circular or elliptical corneal applanation surface S1, and bordering the first upper base surface 4a, 4b without gaps at a second respective straight edge 8a, 8b parallel to the first straight edge 6a, 6b and positioned on an opposite side with respect to the respective first upper base surface 4a, 4b.

As can be clearly seen in Figure 11, also the second straight edge 8a, 8b is at the first distance from the longitudinal axis so that, when the head 1 is viewed exactly in the axial direction Z, the first straight edge 6a, 6b of each prism 3a, 3b appears as the extension of the second straight edge 8b, 8a of the other prism 3b, 3a. When this condition occurs, the ophthalmologist knows that he is looking through the head 1 of the tonometer in a perfectly axial direction, so that it is in the right conditions to carry out the pressure measurements.

As well as the second upper base surface 6a, 6b, also the optional third upper base surface 8a, 8b is configured so that a ray of light entering the end portion 2 from a point on the flat circular or elliptical surface of corneal applanation S1 at a distance from the longitudinal axis Z equal to or greater than the first distance and which is directed along the axial separation plane XZ parallel to the longitudinal axis Z, crosses the end portion 2 and protrudes from the third upper base surface 11c always remaining directed parallel to the longitudinal axis.

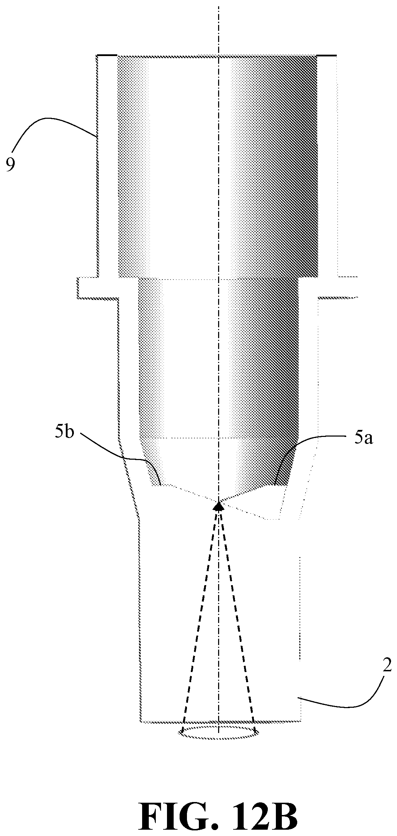

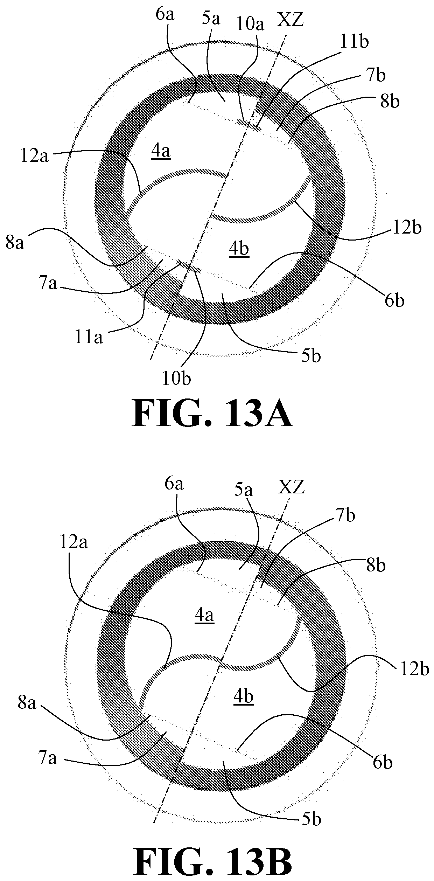

The head 1 according to the present disclosure allows two pressure measurements to be carried out with great accuracy with the Goldmann tonometer: a first measurement is carried out by smoothing a corneal surface of radius $g_1$ equal to the first distance of the edges 6a, 6b from the longitudinal axis, as shown in figure 12A, and a second measurement is performed in the classic Goldmann method, as shown in figure 12B, by smoothing a corneal surface of radius $g_0$ equal to 1.53mm. When the first measurement is carried out (figure 12A), the upper surfaces of the two prisms 3a and 3b divide the luminous contour of the flattened corneal surface of radius $g_1$ in the manner illustrated in figure 13A. This image is obtained only if the flattened corneal surface of radius $g_1$ is perfectly centered with respect to the longitudinal Z axis of the head. In fact, only in this condition, two first light arcs 10a and 10b are formed on the surfaces 5a and 5b of the two prisms 3a and 3b, the light rays of the two arcs enter (figure 12A) in the end portion 2 from the flat corneal applanation surface S1 at a distance from the longitudinal axis Z equal to or greater than the first distance, they propagate parallel to the longitudinal axis Z and emerge from the second upper base surface 5a, 5b always remaining directed parallel to the longitudinal axis Z.

Figures 12A and 13A also show the optional third upper base surface 7a, 7b, also parallel to the flat corneal applanation surface S1, with the related second edge 8a, 8b at the same first distance from the longitudinal axis Z, exactly like the first edge 6a, 6b. Thanks to this optional expedient, two second light arcs 11a and 11b are formed on the third upper base surfaces 7a and 7b of the two prisms 3a and 3b in a completely analogous way to what occurs for the first light arcs 10a and 10b. This optional embodiment is convenient because it allows the centering of the longitudinal axis Z with respect to the flattened corneal surface to be made even more accurate. Indeed, only with perfect centering will the first light arc 10a coming out of the first prism 3a appear as an extension of the second light arc 11b coming out of the second prism 3b, and at the same time the first light arc 10b coming out of the second prism 3b will appear as the extension of the second luminous arc 11a which emerges from the first prism 3a, as shown in figure 13A. Through the first upper base surfaces 4a and 4b two hemicorons 12a and 12b will be seen which do not touch each other, as in figure 6b, because the radius of the flattened corneal surface $g_1$ is greater than the radius $g_0$ for the measurement of the intraocular pressure according to the method of Goldmann.

On the other hand, when the second measurement (figure 12B) is carried out in the classic Goldmann way, the two hemicrons 12a and 12b join in the classic way illustrated in figure 13B. If the flattened corneal surface is centered with respect to the longitudinal axis Z, the arcs through the surfaces 5a, 5b, 7a, 7b will not be seen because the radius $g_0$ is smaller than the first distance of the edges 6a, 6b, 8a, 8b.

An advantage of the tonometer head according to the present disclosure is that it allows for accurate centering of the applanation surface with respect to the apex of the cornea. Currently, the ophthalmologist who carries out the measurement with the Goldmann tonometer believes that the tonometer head is correctly positioned with respect to the corneal apex when the point of contact between the two hemicorons (figure 13B) is more or less in the center of the field of vision. However, this adjustment is approximate and can introduce further errors due to incorrect positioning.

With the tonometer head of the present disclosure, it is first possible to measure the pressure $P_{G1}$ by flattening a corneal surface with a radius equal to $g_1$, until the mirrored arcs 10a and 10b appear with respect to the axial separation plane XZ, which can be performed only if the tonometer head is perfectly centered with respect to the corneal apex, and then the $P_{G0}$ pressure is measured in the classic way by simply reducing the force exerted by the Goldmann tonometer on the cornea without moving it with respect to the previous position.

According to one aspect, the radius $g_1$, i.e. the distance from each of the edges 6a, 6b to the longitudinal axis Z measured along the axial separation plane XZ, is between 1.6 mm and 3 mm, preferably between 1.8 mm and 2.6 mm. Preferably, this radius $g_1$ is equal to 2.0 mm.

In one aspect, the flat corneal applanation surface S1 is circular and has a radius ranging between 3.0 mm and 4.6 mm.

With the applanation head of this disclosure it is possible to carry out a first pressure measurement with the Goldmann tonometer by flattening a corneal surface with a radius equal to $g_1$ (figure 12A), as well as a second standard pressure measurement by smoothing a corneal surface with a radius $g_0$ (smaller than the radius $g_1$) according to the classic Goldmann method. In both measurements, the ophthalmologist has visual reference points which allow him to establish with great accuracy when it is necessary to read the pressure indication on the knob placed on the tonometer.

The possibility of carrying out a pressure measurement when the flattened corneal surface has a radius $g_1$ greater than $g_0$ is an aspect that makes it possible to estimate the corneal elasticity modulus E in vivo, as well as to correct the standard pressure measurement according to the classic Goldmann method removing the disturbance caused by greater or lesser corneal stiffness, thus calculating the "true" intraocular pressure $P_{IOP}$. In order to understand how the methods of the present disclosure allow these excellent results to be achieved with the tonometer head of this disclosure, the results of mathematical studies carried out by the Applicant on the elastic deformation of spherical shells of constant thickness are reported.

Even if globally the cornea is not comparable to a spherical shell of constant thickness, the flattened part during the measurement of the intraocular pressure with the Goldmann tonometer is sufficiently small to be approximated to a spherical shell of practically constant thickness. Theoretical studies carried out by the Applicant on the deformations of elastic spherical shells of constant thickness have shown that the distribution of the pressure exerted by a flattened cornea can be calculated using principles of theory of elastic deformations and that it depends on the central corneal thickness, the central mean radius of curvature of the cornea before applanation, the diameter of the flattened corneal surface, the intraocular fluid pressure (the IOP), and the coefficient of elasticity of the cornea. These theoretical results have been confirmed by laboratory tests carried out by the applicant.

Studies carried out by the Applicant have shown that the overall average pressure exerted by the elastic reaction of the flattened cornea against the head of the Goldmann tonometer alone can be expressed as a function of the central corneal thickness, the central mean radius of curvature of the cornea before applanation, the radius of the flattened corneal surface and the coefficient of elasticity of the cornea. Without thereby being bound by any theory, the Applicant has noted that this average pressure can be approximated as follows:

$$\frac{E \cdot h \cdot g^2}{R^3} \qquad (1)$$

in which

E = Young's modulus averaged over corneal thickness;
h = apical corneal thickness, at the center of the cornea;
g = radius of the flattened corneal area;
R = average radius of curvature (inner radius plus outer radius, divided by two) of the cornea measured in the apical area, i.e. at the center of the cornea.

In the following description, reference will be made to formula (1) for the calculation of the elastic reaction of the flattened cornea only, however what will be said hereinafter remains valid *mutatis mutandis* even if the corneal elastic reaction is estimated with a different formula.

Goldmann established standard reference conditions of central corneal thickness $h_0$, (mean) radius of curvature $R_0$ and modulus of elasticity (Young's modulus) $E_0$ for which the pressure $P_G$ measured on the shaft of his tonometer accurately matched the intraocular pressure $P_{IOP}$. It is commonly believed that the Goldmann tonometer measures intraocular pressure fairly accurately when the cornea has:

- a central corneal thickness $h_0$ equal to 0.536mm;
- a modulus of elasticity $E_0$ (Young's modulus) equal to 0.19MPa;
- an external corneal radius of curvature at the center equal to 7.6 mm;
- an internal corneal radius of curvature at the center equal to 6.7 mm.

This is equivalent to saying that:

$$E_0 = 0.19\text{MPa};$$

$$h_0 = 0.536 \text{ mm};$$

$g_0 = 1.53$ mm is the radius of the applanation area of the Goldmann tonometer;
$R_0 = 7.15$ mm is the mean radius of curvature.

Since the overall average pressure exerted by the elastic reaction of the flattened cornea against the head of the Goldmann tonometer alone, can be estimated approximately with the following ratio:

$$\frac{E \cdot h \cdot g_0^2}{R^3} \qquad (2)$$

then it means that the pressure $P_{G0}$ measured on the rod of the Goldmann tonometer according to the classic Goldmann method, is approximately given by:

$$P_{G0} \cong P_{IOP} + \frac{E \cdot h \cdot g_0^2}{R^3} - \frac{E_0 \cdot h_0 \cdot g_0^2}{R_0^3} \qquad (3)$$

With a tonometer head according to the present disclosure, a new pressure measurement, hereinafter referred to as $P_{G1}$, can be accurately made by flattening a corneal surface having a radius $g_1$ greater than the radius $g_0$ determined by the classic Goldmann method. This radius $g_1$ is equal to half of the reciprocal distance between the straight edges 6a and 6b respectively of the first prism 3a and of the second prism 3b so that, when on the second upper base surface 5a, 5b of each of the two prisms it is possible to see the inner edge of the two hemicrowns (figure 12A), then the longitudinal axis of the tonometer head is perfectly aligned with the corneal apex, because the flattened corneal surface is centered with respect to the corneal apex and has a radius equal to $g_1$. In this new measurement condition, the new measured pressure $P_{G1}$ can be approximated as follows:

$$P_{G1} \cong \frac{g_1^2}{g_0^2} P_{IOP} + \frac{E \cdot h \cdot g_1^4}{R^3 \cdot g_0^2} - \frac{E_0 \cdot h_0 \cdot g_0^2}{R_0^3} \qquad (4)$$

Combining eq.(4) with eq. (3) it is possible to know the value of the "true" $P_{IOP}$ pressure and the value of the corneal modulus of elasticity E:

$$\frac{E \cdot h}{R^3} \cong \frac{1}{g_1^2 - g_0^2} \cdot \left[ \frac{E_0 \cdot h_0 \cdot g_0^2}{R_0^3} \cdot \left( \frac{g_0^2}{g_1^2} - 1 \right) + \frac{g_0^2}{g_1^2} \cdot P_{G1} - P_{G0} \right] \qquad (5)$$

$$P_{IOP} \cong \frac{1}{g_1^2 - g_0^2} \cdot \left[ g_1^2 \cdot P_{G0} - \frac{g_0^4}{g_1^2} \cdot P_{G1} \right] + \frac{E_0 \cdot h_0 \cdot g_0^2}{R_0^3} \cdot \left( \frac{g_0^2}{g_1^2} + 1 \right) \qquad (6)$$

By measuring the central corneal thickness h using any corneal pachymeter and using any keratometer the mean central corneal curvature radius R, knowing the radius $g_0$ of the applanation surface according to the classic method (it is always equal to 1.53 mm) and knowing the radius $g_1$ of the corneal surface flattened with the new measurement according to the present disclosure, the corneal modulus of elasticity E can be easily calculated.

Even if the exact values of the corneal thickness and the mean apical radius of curvature of the cornea are not known, with the aim of knowing the value of the modulus of elasticity only approximately, one can use, instead of the true "h" value and the true "R" value, also corresponding estimated values of them, obtaining in any case an in vivo and reasonably accurate estimate of the value of the modulus of elasticity E. For example, the estimated values for "h" and for "R" may be respective values standard of central corneal thickness $h_0$ and (average) radius of curvature $R_0$ of a typical cornea.

With the applanation head for a Goldmann tonometer according to this disclosure, it is possible to measure the elastic reaction of a flattened cornea by detecting the pressures $P_{G0}$ and $P_{G1}$ measured by the Goldmann tonometer respectively when the cornea is flattened with a radius $g_0$ (classical measurement). and with a radius $g_1$ (new measurement).

Thanks to the double pressure measurement, which may be performed accurately using the tonometer head of this disclosure, it is possible to correct the pressure measurement $P_{G0}$ taken by the Goldmann tonometer according to the classical technique to obtain the "true" $P_{IOP}$ intraocular pressure.

From equation (6) it can be deduced that, by also carrying out the $P_{G1}$ pressure measurement, it is possible to correct the value of the $P_{G0}$ pressure measurement detected by the Goldmann tonometer with the classic technique, to calculate the "true" $P_{IOP}$ intraocular pressure of a patient:

- without knowing the average curvature radius R of the cornea;
- without knowing the modulus of elasticity E of the cornea;
- without knowing the central corneal thickness h.

Consequently, thanks to the tonometer head of the present disclosure it is possible to calculate the "true" intraocular pressure $P_{IOP}$ of a patient without having to use a pachymeter and a keratometer and without resorting to the formulas known in the literature currently used to correct the pressure measurement $P_{G0}$ detected by Goldmann's tonometer, which were obtained heuristically.

Having at one's disposal a Goldmann applanation tonometer and a tonometer head according to the present disclosure, the "true" intraocular pressure $P_{IOP}$ of a patient's eye can be measured by the following operations:

administering an eye dye to a patient's cornea;
detecting, according to Goldmann's classical measurement method, a total pressure $P_{G0}$ exerted by said cornea pressed against the applanation surface of the tonometer head;
detecting a new total pressure $P_{G1}$ exerted by the cornea pressed against the applanation surface of the tonometer head when a circular corneal surface of radius equal to the radius $g_1$ is flattened against the applanation surface S1 of the tonometer head;
calculating the intraocular pressure of the eye on the basis of the pressure $P_{G0}$ and the pressure $P_{G1}$. The latter operation can be carried out for example by using equation (6).

Conveniently, the operations for detecting the new pressure $P_{G1}$ and the pressure $P_{G0}$ can be reversed, so as to accurately center the tonometer head with respect to the corneal apex, before measuring the pressure $P_{G0}$ according to the classic measurement method by Goldmann.

To measure the modulus of elasticity E of a cornea of a patient's eye in vivo using a Goldmann applanation tonometer and a tonometer head according to the present disclosure, the following steps are performed:

measuring a central corneal thickness h and a central curvature radius R of the cornea of the patient's eye;
administering an eye dye to the cornea;
detecting, according to Goldmann's classical measurement method, a total pressure $P_{G0}$ exerted by said cornea pressed against the applanation surface of the tonometer head;
detecting a new overall pressure $P_{G1}$ exerted by the cornea pressed against the applanation surface of the tonometer head when a circular corneal surface of radius equal to the radius $g_1$ is flattened against the applanation surface S1 of the tonometer head;
calculating the corneal modulus of elasticity based on the first pressure $P_{G0}$, on the new pressure $P_{G1}$, on the central corneal thickness h and on the central curvature radius R of the patient's cornea.

According to an aspect not shown in the figures, the second distance $g_2$ of the second straight edges 8a, 8b from the longitudinal axis Z, measured in the axial separation plane XZ, is different from the first distance $g_1$ of the straight edges 6a, 6b, so that the second upper base surfaces 5a, 5b are more distant from the longitudinal axis Z than the third upper base surfaces 7a, 7b. According to one aspect, the opposite is also possible, i.e. that the first distance $g_1$ be greater than the second distance $g_2$.

According to one aspect, the radius $g_2$, i.e. the distance from each of the edges 8a, 8b to the longitudinal axis Z measured along the axial separation plane XZ, is between 1.6 mm and 3 mm, for example between 1.8 mm and 2.6 mm. For example, this radius $g_2$ is equal to 2.5 mm.

According to one aspect, the radius $g_2$ is equal to 2.0 mm and the radius $g_1$ is equal to 2.5 mm. With these values it has been experimentally noted that the estimates obtained for the parameters $E_0$, E and $P_{IOP}$ are less sensitive to imprecisions in the detection of the pressures $P_{G0}$, $P_{G1}$ and $P_{G2}$.

In general, by ensuring that the two distances $g_1$ and $g_2$ are different, it is possible to carry out a third pressure measurement in the same way as shown in figure 12A. If, for example, the second distance $g_2$ is greater than the first distance $g_1$, then it will be necessary to flatten a corneal surface with a radius equal to at least $g_2$ (greater than $g_1$) in order to see a luminous arc respectively through the third upper base surfaces 7a and 7b: when the inner edge of these luminous arcs touches the respective second straight edge 8a and 8b, then the radius of the flattened corneal surface is equal to $g_2$. In this case, the pressure $P_{G2}$ will be given by the following equation:

$$P_{G2} \cong \frac{g_2^2}{g_0^2} P_{IOP} + \frac{E \cdot h \cdot g_2^4}{R^3 \cdot g_0^2} - \frac{E_0 \cdot h_0 \cdot g_0^2}{R_0^3} \qquad (7)$$

This equation, taken together with equations (3) and (4):

$$P_{G1} \cong \frac{g_1^2}{g_0^2} P_{IOP} + \frac{E \cdot h \cdot g_1^4}{R^3 \cdot g_0^2} - \frac{E_0 \cdot h_0 \cdot g_0^2}{R_0^3} \qquad (4)$$

$$P_{G0} \cong P_{IOP} + \frac{E \cdot h \cdot g_0^2}{R^3} - \frac{E_0 \cdot h_0 \cdot g_0^2}{R_0^3} \qquad (3)$$

allows to calculate the "true" intraocular pressure $P_{IOP}$:

$$P_{IOP} \cong \frac{g_0^2 \cdot [P_{G0} \cdot (g_2^4 - g_1^4) + P_{G1} \cdot (g_0^4 - g_2^4) + P_{G2} \cdot (g_1^4 - g_0^4)]}{g_1^2 \cdot g_0^4 + g_0^2 \cdot g_2^4 + g_2^2 \cdot g_1^4 - g_0^2 \cdot g_1^4 - g_1^2 \cdot g_2^4 - g_2^2 \cdot g_0^4} \qquad (8)$$

the modulus of elasticity E:

$$E \cong \frac{R^2}{h} \cdot \frac{g_6^2 \cdot [P_{G0} \cdot (g_1^2 - g_2^2) + P_{G1} \cdot (g_2^2 - g_0^2) + P_{G2} \cdot (g_0^2 - g_1^2)]}{g_1^2 \cdot g_0^4 + g_0^2 \cdot g_2^4 + g_2^2 \cdot g_1^4 - g_0^2 \cdot g_1^4 - g_1^2 \cdot g_0^4 - g_1^2 \cdot g_2^4} \qquad (9)$$

as well as the reference modulus of elasticity $E_0$ of the Goldmann tonometer:

$$E_0 \cong \frac{R_0^3}{h_0 \cdot g_0^2} \cdot \frac{P_{G0} \cdot (g_1^2 \cdot g_0^4 - g_2^2 \cdot g_1^4) + P_{G1} \cdot (g_0^2 \cdot g_2^4 - g_0^2 \cdot g_1^4) + P_{G2} \cdot (g_0^2 \cdot g_1^4 - g_1^2 \cdot g_0^4)}{g_1^2 \cdot g_0^4 + g_0^2 \cdot g_2^4 + g_2^2 \cdot g_1^4 - g_0^2 \cdot g_1^4 - g_1^2 \cdot g_0^4 - g_1^2 \cdot g_2^4} \qquad (10)$$

Accurately knowing the value of the reference modulus of elasticity $E_0$ of a Goldmann tonometer makes it possible to accurately calculate the value of the modulus of elasticity E of a patient's cornea, using the same Goldmann tonometer and a head of the type shown in figures from 7 to 13B, wherein the first edges 6a, 6b are at the same distance as the second edges 8a, 8b from the longitudinal axis Z. Thanks to a tonometer head according to this disclosure with second straight edges 8a, 8b at a distance $g_2$ and first straight edges 6a, 6b at distance $g_1$ from the longitudinal axis Z, eq. (10) allows to check if the reference $E_0$ modulus value of the Goldmann tonometer (on which the head is mounted) has remained the same as the factory value or if it has changed, requiring an operation of calibration of the tonometer. A calibration head can therefore be used with second straight edges 8a, 8b at a distance $g_2$ and first straight edges 6a, 6b at a distance $g_1$ from the longitudinal axis Z, for the preliminary determination of the reference modulus of elasticity $E_0$ of the Goldmann tonometer, in order to then be able to measure more easily the modulus of elasticity E of a patient's cornea by using the head of this disclosure with straight edges 6a, 6b, 8a, 8b equidistant from the longitudinal axis Z.

## Claims

1.    A head for applanation tonometer, comprising a transparent body having a longitudinal axis (Z), defining:
   at a first end along said longitudinal axis (Z), an end portion (2) defining a flat circular or elliptical corneal applanation surface (S1), having a respective radius or semi-axis of applanation, arranged on a first plane of base of the end portion (2) orthogonal to the longitudinal axis (Z) of the tonometer head;
   two semi-cylindrical or semi-elliptical prisms (3a, 3b), each having:

   - a respective lower semicircular or semi-elliptical base integral with said first end (2) and arranged along a second plane orthogonal to said longitudinal axis (Z) and parallel to the flat corneal applanation surface (S1);
   - a first semi-elliptical upper base surface (4a; 4b) opposite said respective lower base and inclined with respect to the lower base by a respective internal angle ($\alpha$) between 8° and 60°;
   said two semi-cylindrical or semi-elliptical prisms (3a, 3b) being opposite along said longitudinal axis (Z) with respect to an axial separation plane (XZ) so that the respective lower semicircular or semi-elliptic bases lie on said second orthogonal plane composing together a full circle or a full ellipse, respectively;
   said end portion (2) and said two prisms (3a, 3b) are integral with each other; **characterized in that**
   each of said two prisms (3a; 3b) defines at least a second upper base surface (5a; 5b) parallel to said flat circular or elliptical corneal applanation surface (S1) and bordering said first upper base surface (4a; 4b ) without interruption at a first straight edge (6a; 6b),
   wherein said first straight edge (6a; 6b) is at a first distance from said longitudinal axis (Z) and said first distance is between 1.6 mm and 3 mm,
   wherein said second upper base surface (5a; 5b) is configured so that a ray of light entering said end portion (2) from a point of the flat circular or elliptical corneal applanation surface (S1) at a distance from the longitudinal axis (Z) equal to or greater than said first distance and which is directed parallel to said longitudinal axis (Z) along said axial separation plane (XZ), crosses said end portion (2) and exits from said second surface of the upper base (5a; 5b) always remaining directed parallel to said longitudinal axis (Z).

2.    The head for applanation tonometer according to claim 1, wherein:
   at least one of said two prisms (3a; 3b) defines at least a third upper base surface (7a; 7b) parallel to said flat circular or elliptical corneal applanation surface (S1) and bordering said first upper base surface (4a; 4b) without interruption at a second straight edge (8a; 8b) parallel to said first straight edge (6a; 6b) and positioned on an opposite side with respect to said respective first upper base surface (4a; 4b), said second straight edge (8a; 8b) is at a second distance from said longitudinal axis, said third upper base surface (7a; 7b) is configured so that a ray of light entering said end portion (2) from a point of the flat circular or elliptical corneal applanation surface (S1) at a distance from longitudinal axis (Z) equal to or greater than said second distance and which is directed parallel to said longitudinal axis (Z) along said axial separation plane (XZ), passes through said end portion (2) and exits from said third base surface upper (7a; 7b) always remaining directed parallel to said longitudinal axis (Z).

3.  The head for applanation tonometer according to claim 2, wherein said second distance is equal to said first distance.

4.  The head for applanation tonometer according to one of claims 2 to 3, wherein said first distance and said second distance are comprised between 1.8 mm and 2.6 mm.

5.  The head for applanation tonometer according to claim 4, wherein said first distance or said second distance is equal to 2.0 mm and said second distance or said first distance is equal to 2.5 mm.

6.  The head for applanation according to one of the preceding claims, wherein said corneal applanation flat surface (S1) is circular and has a radius between 3.0 mm and 4.6 mm.

7.  A method of measuring an intraocular pressure of a patient's eye, comprising the following operations:

    providing a Goldmann applanation tonometer;
    providing and installing a tonometer head according to one of claims 1 to 6;
    administering an ophthalmic dye to a patient's cornea;
    detecting, according to a Goldmann method of measuring an intraocular pressure, a first overall pressure exerted by said cornea flattened against the applanation surface of the tonometer head;
    detecting a second overall pressure exerted by the cornea flattened against the applanation surface of the tonometer head when a circular corneal surface having a radius equal to said first distance is flattened against the applanation surface;
    calculating the intraocular pressure of the eye on the basis of said first pressure and of said second pressure.

8.  A method of in vivo measurement of a modulus of elasticity of a cornea of a patient's eye, comprising the following operations:

    providing a Goldmann applanation tonometer;
    providing and installing a tonometer head according to one of claims 1 to 6;
    measuring a central corneal thickness and a central curvature radius of the cornea of said patient's eye;
    administering an ophthalmic dye on said cornea;
    detecting, according to a Goldmann method of measuring intraocular pressure, a first overall pressure exerted by the cornea pressed against the applanation surface of the tonometer head;
    detecting a second overall pressure exerted by the cornea flattened against the applanation surface of the tonometer head when a circular corneal surface having a radius equal to said first distance is flattened against the applanation surface;
    calculating the modulus of elasticity of the cornea on the basis of said first pressure, of said second pressure, of said central corneal thickness and of said central radius of curvature.

9.  The method according to claim 8. comprising the step of determining said central corneal radius of curvature as an intermediate value between its inner central radius of curvature and its outer central radius of curvature.

10. The method according to claim 8 or 9, comprising the operations of:

    providing and installing a tonometer head according to one of claims 2, 4 or 5;
    detecting a third overall pressure exerted by the cornea pressed against the applanation surface of the tonometer head when a circular corneal surface having a radius equal to said second distance is flattened against the applanation surface;
    calculating the modulus of elasticity of the cornea on the basis of said first pressure, of said second pressure, of said third pressure, of said central corneal thickness and of said central radius of curvature.

**Patentansprüche**

1.  Kopf für ein Applanationstonometer, umfassend einen transparenten Körper mit einer Längsachse (Z), definierend:

an einem ersten Ende entlang der Längsachse (Z), einen Endabschnitt (2), der eine flache, kreisförmige oder elliptische Hornhaut-Applanationsfläche (S1) mit einem entsprechenden Radius oder einer entsprechenden Halbachse der Applanation definiert, die auf einer ersten Basisebene des Endabschnitts (2) orthogonal zur Längsachse (Z) des Tonometerkopfes angeordnet ist;

zwei halbzylindrische oder halbelliptische Prismen (3a, 3b), jeweils aufweisend:

- eine entsprechende untere halbkreisförmige oder halbelliptische Basis, die integral mit dem ersten Ende (2) ist und entlang einer zweiten Ebene orthogonal zu der Längsachse (Z) und parallel zu der flachen Hornhaut-Applanationsfläche (S1) angeordnet ist;
- eine erste halbelliptische obere Basisoberfläche (4a; 4b), die der jeweiligen unteren Basis gegenüberliegt und in Bezug auf die untere Basis um einen jeweiligen inneren Winkel ($\alpha$) zwischen 8° und 60° geneigt ist;

wobei die beiden halbzylindrischen oder halbelliptischen Prismen (3a, 3b) entlang der Längsachse (Z) in Bezug auf eine axiale Trennebene (XZ) einander gegenüberliegen, so dass die jeweiligen unteren halbkreisförmigen oder halbelliptischen Basen auf der zweiten orthogonalen Ebene liegen, die zusammen einen Vollkreis bzw. eine Vollellipse bilden;

der Endabschnitt (2) und die zwei Prismen (3a, 3b) sind integral miteinander verbunden;

**dadurch gekennzeichnet, dass**

jedes der beiden Prismen (3a; 3b) mindestens eine zweite obere Basisfläche (5a; 5b) parallel zur flachen, kreisförmigen oder elliptischen Hornhaut-Applanationsfläche (S1) definiert und ohne Unterbrechung an einer ersten geraden Kante (6a; 6b) an die erste obere Basisfläche (4a; 4b) angrenzt,

wobei die erste gerade Kante (6a; 6b) einen ersten Abstand von der Längsachse (Z) aufweist und dieser erste Abstand zwischen 1,6 mm und 3 mm beträgt,

wobei die zweite obere Basisfläche (5a; 5b) so konfiguriert ist, dass ein Lichtstrahl, der von einem Punkt der flachen, kreisförmigen oder elliptischen Hornhaut-Applanationsfläche (S1) in den Endabschnitt (2) eintritt, der einen Abstand von der Längsachse (Z) aufweist, der gleich oder größer als der erste Abstand ist und der parallel zu der Längsachse (Z) entlang der axialen Trennebene (XZ) gerichtet ist, den Endabschnitt (2) kreuzt und aus der zweiten Fläche der oberen Basis (5a; 5b) austritt, wobei er immer parallel zu der Längsachse (Z) gerichtet bleibt.

2. Kopf für ein Applanationstonometer nach Anspruch 1, wobei:
mindestens eines der beiden Prismen (3a; 3b) mindestens eine dritte obere Basisfläche (7a; 7b) parallel zur flachen, kreisförmigen oder elliptischen Hornhaut-Applanationsfläche (S1) definiert und ohne Unterbrechung an die erste obere Basisfläche (4a; 4b) angrenzt, wobei eine zweite gerade Kante (8a; 8b) parallel zur ersten geraden Kante (6a; 6b) verläuft und sich auf der gegenüberliegenden Seite der jeweiligen ersten oberen Basisfläche (4a; 4b) befindet, wobei die zweite gerade Kante (8a; 8b) einen zweiten Abstand von der Längsachse hat, wobei die dritte obere Basisfläche (7a; 7b) so konfiguriert ist, dass ein Lichtstrahl, der von einem Punkt der flachen, kreisförmigen oder elliptischen Hornhaut-Applanationsfläche (S1) in einem Abstand von der Längsachse (Z), der gleich oder größer als der zweite Abstand ist, in den Endabschnitt (2) eintritt und parallel zur Längsachse (Z) entlang der axialen Trennebene (XZ) gerichtet ist, durch den Endabschnitt (2) verläuft und von der dritten oberen Basisfläche (7a; 7b) austritt, wobei er immer parallel zur Längsachse (Z) ausgerichtet bleibt.

3. Kopf für Applanationstonometer nach Anspruch 2, wobei der zweite Abstand gleich dem ersten Abstand ist.

4. Kopf für Applanationstonometer nach einem der Ansprüche 2 bis 3, wobei der erste Abstand und der zweite Abstand jeweils einen Bereich zwischen 1,8 mm und 2,6 mm umfassen.

5. Kopf für Applanationstonometer nach Anspruch 4, wobei der erste Abstand oder der zweite Abstand 2,0 mm und der zweite Abstand oder der erste Abstand 2,5 mm beträgt.

6. Kopf für Applanationstonometer nach einem der vorstehenden Ansprüche, wobei die flache Hornhaut-Applanationsfläche (S1) kreisförmig ist und einen Radius zwischen 3,0 mm und 4,6 mm aufweist.

7. Verfahren zum Messen des Augeninnendrucks eines Patientenauges, das die folgenden Vorgänge umfasst:

Bereitstellen eines Goldmann-Applanationstonometers;
Bereitstellen und Installieren eines Tonometerkopfes nach einem der Ansprüche 1 bis 6;
Verabreichen eines ophthalmischen Farbstoffs auf die Hornhaut eines Patienten;
Erfassen, gemäß eines Goldmann-Verfahrens zur Messung des Augeninnendrucks, eines ersten Gesamtdrucks, der von der gegen die Applanationsfläche des Tonometerkopfes abgeflachten Hornhaut ausgeübt wird;

Erfassen eines zweiten Gesamtdrucks, der von der gegen die Applanationsfläche des Tonometerkopfes abgeflachten Hornhaut ausgeübt wird, wenn eine kreisförmige Hornhautoberfläche mit einem Radius gleich dem ersten Abstand gegen die Applanationsfläche abgeflacht wird;

Berechnen des Augeninnendrucks des Auges auf der Grundlage des ersten Drucks und des zweiten Drucks.

8. Verfahren zur In-vivo-Messung eines Elastizitätsmoduls einer Hornhaut des Auges eines Patienten, die folgenden Vorgänge umfassend:

Bereitstellen eines Goldmann-Applanationstonometers;

Bereitstellen und Installieren eines Tonometerkopfes nach einem der Ansprüche 1 bis 6;

Messen einer zentralen Hornhautdicke und eines zentralen Krümmungsradius der Hornhaut des Auges des Patienten;

Verabreichen eines ophthalmischen Farbstoffs auf die Hornhaut;

Erfassen, gemäß eines Goldmann-Verfahrens zur Messung des Augeninnendrucks, eines ersten Gesamtdrucks, der von der gegen die Applanationsfläche des Tonometerkopfes gedrückten Hornhaut ausgeübt wird;

Erfassen eines zweiten Gesamtdrucks, der von der gegen die Applanationsfläche des Tonometerkopfes abgeflachten Hornhaut ausgeübt wird, wenn eine kreisförmige Hornhautoberfläche mit einem Radius gleich dem ersten Abstand gegen die Applanationsfläche abgeflacht wird;

Berechnen des Elastizitätsmoduls der Hornhaut auf der Grundlage des ersten Drucks, des zweiten Drucks, der zentralen Hornhautdicke und des zentralen Krümmungsradius.

9. Verfahren nach Anspruch 8, umfassend den Schritt des Bestimmens des zentralen Krümmungsradius der Hornhaut als Zwischenwert zwischen ihrem inneren zentralen Krümmungsradius und ihrem äußeren zentralen Krümmungsradius.

10. Verfahren nach Anspruch 8 oder 9, umfassend die Vorgänge:

Bereitstellen und Installieren eines Tonometerkopfes nach einem der Ansprüche 2, 4 oder 5;

Erfassen eines dritten Gesamtdrucks, der von der gegen die Applanationsfläche des Tonometerkopfes gedrückten Hornhaut ausgeübt wird, wenn eine kreisförmige Hornhautoberfläche mit einem Radius gleich dem zweiten Abstand gegen die Applanationsfläche abgeflacht wird;

Berechnen des Elastizitätsmoduls der Hornhaut auf der Grundlage des ersten Drucks, des zweiten Drucks, des dritten Drucks, der zentralen Hornhautdicke und des zentralen Krümmungsradius.

## Revendications

1. Tête pour tonomètre à aplanation, comprenant un corps transparent ayant un axe longitudinal (Z), définissant :

à une première extrémité le long dudit axe longitudinal (Z), une partie d'extrémité (2) définissant une surface d'aplanation de la cornée plane, circulaire ou elliptique (S1), ayant un rayon ou un demi-axe d'aplanation respectif, disposé sur un premier plan de base de la partie d'extrémité (2) orthogonal à l'axe longitudinal (Z) de la tête de tonomètre ;

deux prismes (3a, 3b) semi-cylindriques ou semi-elliptiques, chacun ayant :

- une base inférieure semi-circulaire ou semi-elliptique respective solidaire de ladite première extrémité (2) et disposée le long d'un second plan orthogonal audit axe longitudinal (Z) et parallèle à la surface d'aplanation de la cornée plane (S1) ;

- une première surface de base supérieure semi-elliptique (4a ; 4b) opposée à ladite base inférieure respective et inclinée par rapport à la base inférieure d'un angle interne ($\alpha$) respectif compris entre 8° et 60°;

lesdits deux prismes (3a, 4b) semi-cylindriques ou semi-elliptiques étant opposés le long dudit axe longitudinal (Z) par rapport à un plan de séparation axial (XZ), de sorte que les bases inférieures semi-circulaires ou semi-elliptiques respectives se trouvent sur ledit second plan orthogonal, composant ensemble un cercle complet ou une ellipse complète, respectivement ;

ladite partie d'extrémité (2) et lesdits deux prismes (3a, 4b) sont solidaires les uns des autres ;

**caractérisée en ce que**

chacun desdits deux prismes (3a ; 3b) définit au moins une deuxième surface de base supérieure (5a ; 5b) parallèle à ladite surface d'aplanation de la cornée plane, circulaire ou elliptique (S1) et bordant ladite première surface de base supérieure (4a ; 4b) sans interruption au niveau d'un premier bord droit (6a ; 6b),

dans laquelle ledit premier bord droit (6a ; 6b) est situé à une première distance dudit axe longitudinal (Z) et ladite première distance est comprise entre 1,6 mm et 3 mm,

dans laquelle ladite deuxième surface de base supérieure (5a ; 5b) est conçue de sorte qu'un rayon de lumière entrant dans ladite partie d'extrémité (2) à partir d'un point de la surface d'aplanation de la cornée plane, circulaire ou elliptique (S1) à une distance de l'axe longitudinal (Z) égale ou supérieure à ladite première distance et qui est dirigé parallèlement audit axe longitudinal (Z) le long dudit plan de séparation axial (XZ) traverse ladite partie d'extrémité (2) et sort de ladite deuxième surface de la base supérieure (5a ; 5b) en restant toujours dirigé parallèlement audit axe longitudinal (Z).

2. Tête pour tonomètre à aplanation selon la revendication 1, dans laquelle :

au moins l'un desdits deux prismes (3a ; 3b) définit au moins une troisième surface de base supérieure (7a ; 7b) parallèle à ladite surface d'aplanation de la cornée plane, circulaire ou elliptique (S1) et bordant ladite première surface de base supérieure (4a ; 4b) sans interruption au niveau d'un second bord droit (8a ; 8b) parallèle audit premier bord droit (6a ; 6b) et positionnée sur un côté opposé par rapport à ladite première surface de base supérieure (4a ; 4b) respective, ledit second bord droit (8a ; 8b) est situé à une seconde distance dudit axe longitudinal, ladite troisième surface de base supérieure (7a ; 7b) est conçue de sorte qu'un rayon de lumière entrant dans ladite partie d'extrémité (2) à partir d'un point de la surface d'aplanation de la cornée plane, circulaire ou elliptique (S1) à une distance de l'axe longitudinal (Z) égale ou supérieure à ladite seconde distance et qui est dirigé parallèlement audit axe longitudinal (Z) le long dudit plan de séparation axial (XZ) traverse ladite partie d'extrémité (2) et sort de ladite troisième surface de base supérieure (7a ; 7b) en restant toujours dirigé parallèlement audit axe longitudinal (Z).

3. Tête pour tonomètre à aplanation selon la revendication 2, dans laquelle ladite seconde distance est égale à ladite première distance.

4. Tête pour tonomètre à aplanation selon l'une des revendications 2 à 3, dans laquelle ladite première distance et ladite seconde distance sont comprises entre 1,8 mm et 2,6 mm.

5. Tête pour tonomètre à aplanation selon la revendication 4, dans laquelle ladite première distance ou ladite seconde distance est égale à 2,0 mm et ladite seconde distance ou ladite première distance est égale à 2,5 mm.

6. Tête pour tonomètre à aplanation selon l'une des revendications précédentes, dans laquelle ladite surface d'aplanation de la cornée plane (S1) est circulaire et a un rayon compris entre 3,0 mm et 4,6 mm.

7. Procédé pour la mesure d'une pression intraoculaire de l'œil d'un patient, comprenant les étapes suivantes :

fourniture d'un tonomètre à aplanation de Goldmann ;
fourniture et installation d'une tête de tonomètre selon l'une des revendications 1 à 6 ; administration d'un colorant ophtalmique sur la cornée d'un patient ;
détection, selon un procédé de Goldmann de mesure de la pression intraoculaire, d'une première pression globale exercée par ladite cornée aplatie contre la surface d'aplanation de la tête de tonomètre ;
détection d'une deuxième pression globale exercée par la cornée aplatie contre la surface d'aplanation de la tête de tonomètre lorsqu'une surface circulaire de la cornée ayant un rayon égal à ladite première distance est aplatie contre la surface d'aplanation ;
calcul de la pression intraoculaire de l'œil sur la base de ladite première pression et de ladite deuxième pression.

8. Procédé pour la mesure *in vivo* du module d'élasticité de la cornée de l'œil d'un patient, comprenant les étapes suivantes :

fourniture d'un tonomètre à aplanation de Goldmann ;
fourniture et installation d'une tête de tonomètre selon l'une des revendications 1 à 6 ;
mesure de l'épaisseur centrale de la cornée et du rayon de courbure central de la cornée dudit œil du patient ;
administration d'un colorant ophtalmique sur ladite cornée ;
détection, selon un procédé de Goldmann de mesure de la pression intraoculaire, d'une première pression globale exercée par la cornée aplatie contre la surface d'aplanation de la tête de tonomètre ;
détection d'une deuxième pression globale exercée par la cornée aplatie contre la surface d'aplanation de la tête de tonomètre lorsqu'une surface circulaire de la cornée ayant un rayon égal à ladite première distance est aplatie contre la surface d'aplanation ;

calcul du module d'élasticité de la cornée sur la base de ladite première pression, de ladite deuxième pression, de ladite épaisseur centrale de la cornée et dudit rayon de courbure central.

9. Procédé selon la revendication 8, comprenant l'étape consistant à déterminer ledit rayon de courbure central de la cornée comme valeur intermédiaire entre son rayon de courbure central interne et son rayon de courbure central externe.

10. Procédé selon la revendication 8 ou 9, comprenant les étapes consistant à :

fournir et installer une tête de tonomètre selon l'une des revendications 2, 4 ou 5 ;

détecter une troisième pression globale exercée par la cornée pressée contre la surface d'aplanation de la tête de tonomètre lorsqu'une surface circulaire de la cornée ayant un rayon égal à ladite seconde distance est aplatie contre la surface d'aplanation ;

calculer le module d'élasticité de la cornée sur la base de ladite première pression, de ladite deuxième pression, de ladite troisième pression, de ladite épaisseur centrale de la cornée et dudit rayon de courbure central.

**FIG. 1**

**FIG. 2**

**FIG. 3**

**FIG. 4**

**FIG. 5**

**FIG. 6**

**FIG. 7**

**FIG. 8**

**FIG. 9**

3a

6a          5a

7b

8b

4a          3b

8a          4b

7a

6b

5b

# FIG. 10

6a     5a     XZ

7b

8b

4a

8a          4b

6b

7a

5b

# FIG. 11

**FIG. 12A**

**FIG. 12B**

y

y

**FIG. 13A**

**FIG. 13B**

**EP 4 463 056 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021205310 A

- US 5305747 A